# FASCICULE DE BREVET EUROPEEN

(11) **EP 0 563 219 B1**
(45) Date de publication et mention de la délivrance du brevet: **30.11.1994**
(21) Numéro de dépôt: 92902174.9
(22) Date de dépôt: 17.12.1991
(51) Int. Cl.: C07D 275/06

(54) **PROCEDE DE PREPARATION DE DERIVES "SYN" DU PROPANAMIDE**
VERFAHREN ZUR HERSTELLUNG VON PROPANAMID "SYN"-DERIVATEN
METHOD OF PREPARING "SYN" DERIVATIVES OF PROPANAMIDE

(30) Priorité: 18.12.1990 FR 9015814
(43) Date de publication de la demande: 06.10.1993
(73) Titulaire: RHONE-POULENC RORER S.A., 92160 Antony (FR)
(72) Inventeur: AUBERT, Thierry, F-64000 PAU (FR); RADISSON, Xavier, F-69003 Lyon (FR)
(74) Mandataire: Pilard, Jacques
(86) Numéro de dépôt international: FR9101019
(87) Numéro de publication internationale: WO9211250

(56) Documents cités:
- CHEMICAL ABSTRACTS, vol.112, no. 23, 4 June 1990, Columbus Ohio,US,
- FIESER & FIESERS - REAGENT FOR ORGANIC SYNTHESIS, vol.12,1986
- FIESER & FIESERS - REAGENT FOR ORGANIC SYNTHESIS, vol. 13 1988
- THEILHEIMERS SYNTHETIC METHODS OF ORGANIC CHEMISTRY vol. 38, 1984
- CHEMICAL ABSTRACTS, vol. 113, No. 17, 22 October 1990, Columbus OhioUS , W. OPPOLZER ET AT

## Description

La présente invention concerne un procédé de préparation d'un dérivé "syn" du propanamide de foule générale : 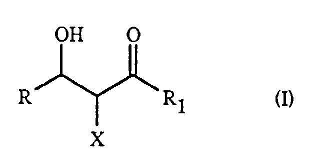
dans laquelle :
X représente un atome d'halogène, de préférence un atome de chlore ou de brome
R représente un radical alkyle, aryle ou hétéroaryle, et
R₁ représente le reste de l'un ou l'autre des énantiomères du camphosultame de formule : 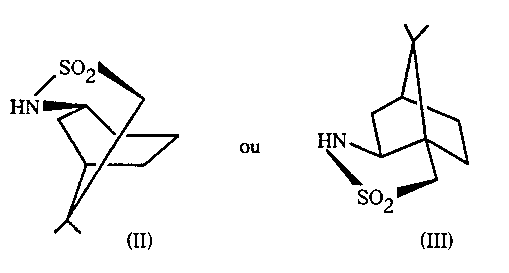
étant entendu que, selon la nature de R₁, les dérivés "syn" du propanamide de formule générale (I) ont la configuration suivante : 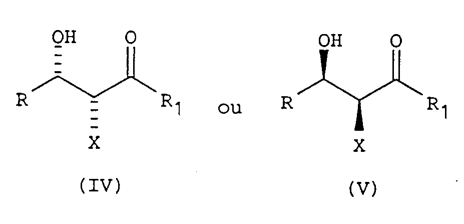
D'un intérêt tout particulier sont les produits de formule générale (I) dans laquelle R représente un radical phényle ou un radical (trityl-1 1H-imidazolyl-4).

Dans J. Amer. Chem. Soc., 112, 2767-2772 (1990), W. Oppolzer et coll. enseignent que des acylsultames traités par un aldéhyde, en présence d'un dérivé du bore, conduisent aux aldols syn de formule : 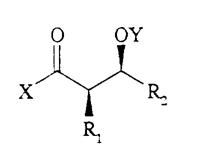
alors que, en présence d'un dérivé de l'étain, ils conduisent aux aldols syn de formule : 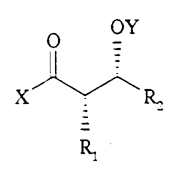
J. Amer. Chem. Soc., 112, 2767-2772 (1990) (D1) n'enseigne pas la possibilité de mettre en oeuvre le procédé en présence d'un sel de titane.

Teilheimer's synthetic methods of organic chemistry, 38, 255 (1984) enseigne que le traitement de la bromoacétone par un aldéhyde en présence d'un dérivé de l'étain conduit à un aldol de formule : 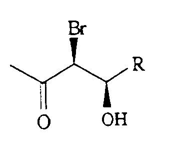
Il est connu d'après J. Amer. Chem. Soc., 112, 866-868 (1990) qu'un produit de formule : 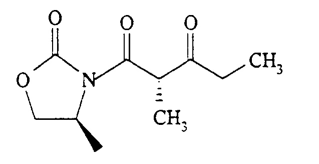
traité par un aldéhyde aromatique en présence d'un sel de titane et d'une amine tertiaire conduit sélectivement au dérivé syn de formule : 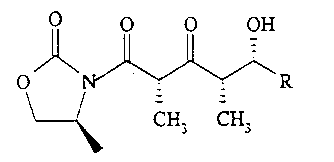
le rapport des isomères syn étant 96/4.

Il est connu également, d'après T. Owa et coll., Chemistry Letters, 1873-1874 (1988) que la (R)-3-bromoacetyl-4-isopropyl-1,3-oxazolidine-2-one réagit avec le 1-triphénylméthylimidazole-4-carbaldéhyde pour donner le dérivé syn de formule :

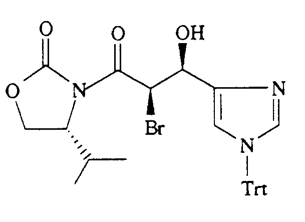

Selon l'invention, les produits de formule générale (I) sont obtenus par condensation d'un dérivé du camphosultame de formule générale : 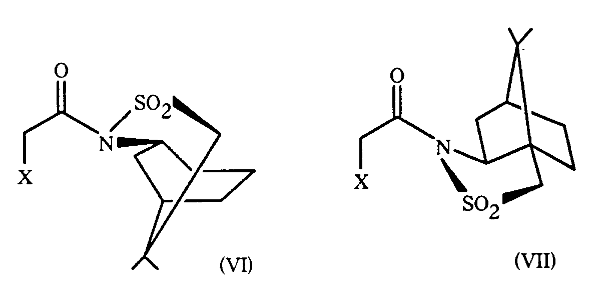
dans laquelle X est défini comme précédemment, sur un aldéhyde de formule générale :

R-CHO (VIII)

dans laquelle R est défini comme précédemment, en présence de chlorure de titane (TiCl₄) et d'une base organique en opérant dans un solvant organique inerte à une température comprise entre -50 et +50°C et de préférence entre -30 et 0°C.

Généralement, on utilise de 0,3 à 3 moles de chlorure de titane par mole de produit de formule générale (VI) ou (VII), de préférence 1 mole.

Comme bases organiques peuvent être utilisées les amines tertiaires aliphatiques telles que la diisopropyléthylamine ou la triéthylamine. De préférence, on utilise la diisopropyléthylamine.

Généralement, on utilise au moins une mole de base par mole de produit de formule générale (VI) ou (VII).

Comme solvant organique inerte, il est particulièrement avantageux d'utiliser un hydrocarbure aliphatique halogéné tel que le chlorure de méthylène.

Selon le dérivé du camphosultame de formule (VI) ou (VII) utilisé et la nature de l'aldéhyde de formule générale (VIII), on obtient le dérivé "syn" de formule (IV) ou (V) éventuellement associé au dérivé "anti" correspondant. Pour un aldéhyde de formule (VIII) déterminé, si l'une des formes (VI) ou (VII) du dérivé du camphosultame conduit à l'une des formes (IV) ou (V) du produit de formule générale (I), l'autre forme du dérivé du camphosultame conduit nécessairement à l'autre forme (IV) ou (V) du produit de formule générale (I).

Le dérivé "syn" pratiquement pur peut être obtenu à partir de son mélange avec le dérivé "anti" par recristallisation dans un solvant approprié.

Le produit de formule générale (VI) ou (VII) peut être obtenu par action d'un halogénure d'un acide halogénoacétique sur un camphosultame de formule (II) ou (III) dans les conditions décrites par W. Oppolzer et al., J. Amer. Chem. Soc., 112, 2767-2772 (1990).

Le produit de formule générale (I) sous forme (IV) dans laquelle X représente un atome d'halogène, de préférence un atome de brome, R représente un radical phényle est particulièrement utile pour préparer le taxol ou ses dérivés de formule générale : 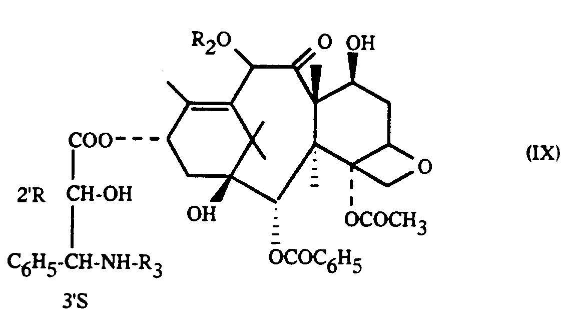
dans laquelle R₂ représente un atome d'hydrogène ou un radical acétyle et R₃ représente un radical benzoyle ou t.butoxycarbonyle qui présentent des propriétés antitumorales remarquables.

Les produits de formule générale (IX) dans laquelle R₂ représente un atome d'hydrogène ou un radical acétyle et R₃ représente un radical benzoyle sont respectivement le désacétyl-10 taxol et le taxol.

Les produits de formule générale (IX) dans laquelle R₂ représente un atome d'hydrogène ou un radical acétyle et R₃ représente un radical t.butoxycarbonyle sont décrits dans le brevet européen EP-B-0253738.

Le produit de formule générale (I) sous forme (IV), dans laquelle R représente un radical phényle et X représente de préférence un atome de brome, traité par une base telle que le méthylate de sodium ou le benzylate de lithium, conduit à un ester de l'acide cis-β-phénylglycidique de formule générale : 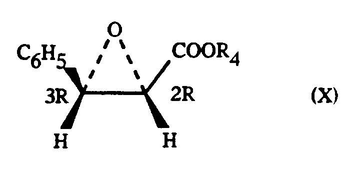
dans laquelle R₄ représente un radical alcoyle (méthyle) ou aralcoyle (benzyle).

Le produit de formule générale (X), par action d'un azoture, conduit au produit de formule générale : 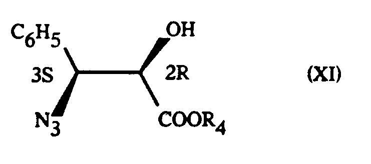
dans laquelle R₄ est défini comme précédemment, dont la fonction hydroxy est ensuite protégée par un groupement R₅de façon à obtenir un produit de formule générale : 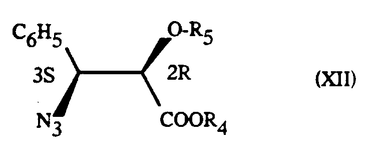
dans laquelle R₄ est défini comme précédemment et R₅représente un groupement protecteur de la fonction hydroxy, qui est réduit en produit de formule générale : 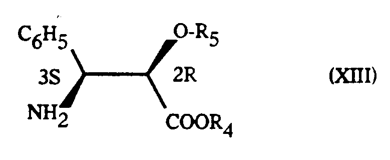
dans laquelle R₄ et R₅ sont définis comme précédemment, qui est traité par un réactif permettant d'introduire un groupement benzoyle ou t.butoxycarbonyle pour obtenir un produit de formule générale : 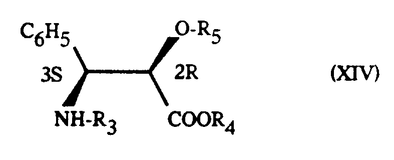
dans laquelle R₃, R₄ et R₅ sont définis comme précédemment qui est ensuite saponifié pour donner un produit de formule générale : 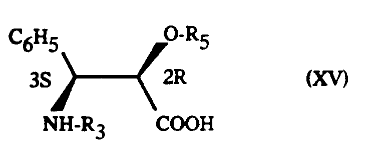
qui est condensé sur la baccatine III ou la désacétyl-10 baccatine III dont les fonctions hydroxy en -7 et éventuellement en -10 sont protégées par un groupement protecteur choisi parmi les radicaux trichloro-2,2,2 éthoxycarbonyle ou trialkylsilyle, pour donner un produit de formule générale (IX) après remplacement des groupements protecteurs présents par des atomes d'hydrogène.

Le produit de formule générale (I) sous forme (IV) dans laquelle R représente le radical trityl-1 1H-imidazolyl-4 et X représente un atome de chlore, est particulièrement utile pour préparer l'amino-3 (amino-2 1H-imidazolyl-4)-1 chloro-2 propanol-1- (1S,2S) qui est décrit dans le brevet européen EP-B-0215687 et qui présente d'intéressantes propriétés antitumorales.

Par action de l'ammoniac sur le produit de formule générale (I) sous forme (IV) dans laquelle R représente un radical trityl-1 1H-imidazolyl-4 et X représente un atome de chlore, est obtenu le dérivé de l'imidazolepropanamide de formule : 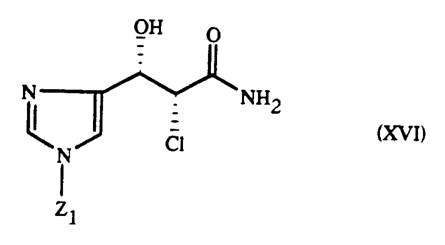
dans laquelle Z₁ est défini comme précédemment.

Par réduction, au moyen par exemple d'un hydrure de bore, du produit de formule (XVI) est obtenu le produit de formule : 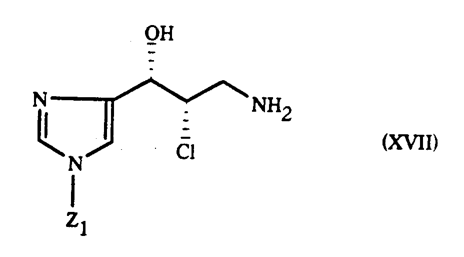
dans laquelle Z₁ représente le radical trityle.

Par action d'un agent de blocage de la fonction amine sur le produit (XVII) est obtenu le produit de formule générale : 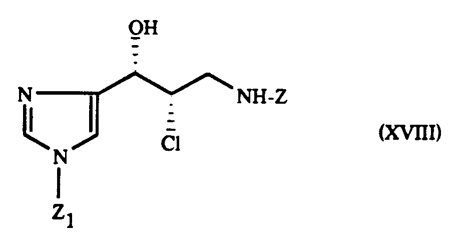
dans laquelle Z₁ est défini comme précédemment et Z représente un groupement protecteur de la fonction amine facilement remplaçable par un atome d'hydrogène par hydrolyse en milieu acide.

Par élimination sélective du groupement protecteur Z₁ du produit de formule générale (XVIII) est obtenu le produit de formule générale : 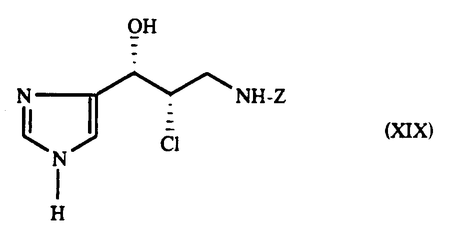
dans laquelle Z est défini comme précédemment.

Par diazotation du produit de formule générale (XIX) au moyen d'un sel de diazonium est obtenu le produit de formule générale: 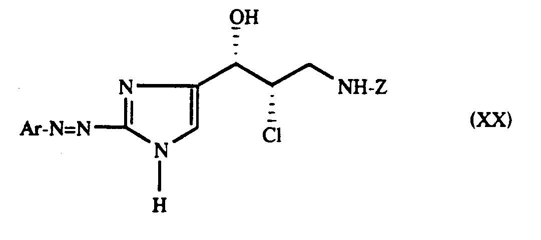
dans laquelle Ar représente un radical phényle éventuellement substitué et Z est défini comme précédemment.

Par réduction, au moyen d'hydrogène en présence d'un catalyseur, du produit de formule générale (XX) est obtenu le produit de formule générale : 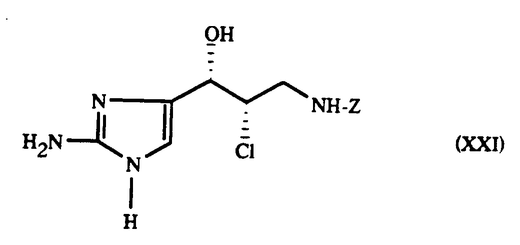
dans laquelle Z est défini comme précédemment.

Par remplacement du groupement protecteur Z du produit de formule générale (XXI) par un atome d'hydrogène, est obtenu l'amino-3 (amino-2' 1H-imidazolyl-4')-1 chloro-2 propanol-1-(1S,2S) qui peut être isolé éventuellement sous forme de sel.

Le produit de formule générale (I) dans laquelle X représente un atome de brome et R représente un radical trityl-1 1H-imidazolyl-4 peut être utilisé pour préparer l'érythro-β-hydroxy-L-histidine qui est un acide aminé constituant des bléomycines dans les conditions décrites par T. OWA et al, Chemistry Letters, 1873 (1988).

Les exemples suivants, donnés à titre non limitatif, montrent comment l'invention peut être mise en pratique.

### EXEMPLE 1

Dans un ballon de 100 cm3, on introduit, sous atmosphère d'argon, 1,55 g de (-)-N-chloracétyl bornanesultame-10,2 (5,31 mmoles) dans 20 cm3 de dichlorométhane. On refroidit à -30°C puis on ajoute, en 5 minutes, 0,584 cm3 de chlorure de titane (5,31 mmoles). Après 30 minutes d'agitation, on obtient une solution jaune clair.

On ajoute alors, à -30°C, en 10 minutes, 0,925 cm3 de diisopropyléthylamine (5,31 mmoles). Le mélange réactionnel devient rouge. On agite pendant 30 minutes à -30°C.

On ajoute ensuite, à -30°C, en 10 minutes, 0,486 cm3 de benzaldéhyde (4,78 mmoles). On agite pendant 30 minutes à -30°C puis on laisse remonter la température au voisinage de 0°C et agite à nouveau pendant 45 minutes à cette température. On obtient une solution jaune orange.

On ajoute 1,1 cm3 d'une solution tampon à pH=7. Après décantation, la phase organique est lavée puis séchée sur sulfate de sodium. Après filtration et concentration à sec, on obtient 1,71 g d'un solide blanchâtre.

Après recristallisation dans 40 cm3 d'acétate d'éthyle bouillant, on obtient 0,959 g de N-[hydroxy-3' chloro-2' phényl-3' propanoyl] bornanesultame-10,2-(2'R,3'S).

Le rendement est de 50,4 %.

### EXEMPLE 2

Dans un ballon tricol de 500 cm3, on introduit à -30°C sous atmosphère d'argon 19,8 g de (-)-N-chloracétyl bornanesultame-10,2 (67,9 mmoles) en solution dans 200 cm3 de dichlorométhane. On ajoute, en 10 minutes, à une température comprise entre -31 et -33°C, 66 cm3 de chlorure de titane en solution 1M dans le dichlorométhane. On agite pendant 30 minutes.

On ajoute alors en 10 minutes, à -30°C, 11,8 cm3 de diisopropyléthylamine. On agite à nouveau pendant 30 minutes à cette température.

Au mélange réactionnel noir, on ajoute, en 10 minutes, 22,95 g de trityl-1 imidazolecarboxaldéhyde-4 dans 75 cm3 de dichlorométhane.

Après 45 minutes d'agitation, on ajoute, à 0°C, 100 cm3 d'une solution tampon à pH=7.

Après décantation, la phase organique est lavée par 60 cm3 d'une solution saturée de bicarbonate, par 2 fois 100 cm3 d'eau puis séchée sur sulfate de magnésium. Après filtration et évaporation à sec, on obtient, avec un rendement de 95 %, 39,36 g d'un produit, sous forme de meringue, dont l'analyse montre qu'il contient 71 % d'isomère "syn" (2'R,3'S), 12 % d'isomère "syn" (2'S,3'R) et 17 % d'isomère "anti" (2'S,3'S).

Par recristallisation dans 1140 cm3 d'acétate d'éthyle, on obtient 18,13 g de N-[hydroxy-3' chloro-2' (trityl-1'' 1H-imidazolyl-4'')-3' propanoyl] bornanesultame-10,2-(2'R,3'S) sous forme de cristaux blancs.

L'analyse par résonance magnétique nucléaire du proton montre qu'il contient plus de 95 % d'isomère "syn" (2'R,3'S).

### EXEMPLE 3

Dans un ballon de 250 cm3, on introduit à -30°C, sous atmosphère d'argon, 1,5 g de (+)-N-chloracétyl bornanesultame-10,2 (5,14 mmoles) a solution dans 20 cm3 de dichlorométhane. On ajoute, en 10 minutes, à une température voisine de -30°C, 0,57 cm3 de chlorure de titane (5,14 mmoles). On agite pendant 30 minutes puis on ajoute, à -30°C, 0,9 cm3 de diisopropyléthylamine (5,14 mmoles). On agite pendant 30 minutes à -30°C puis on ajoute 0,44 cm3 de pyridinecarboxaldéhyde-2 (4,61 mmoles). On agite pendant 1 heure à -30°C puis laisse remonter la température au voisinage de 0°C.

On ajoute 10 cm3 de tampon à pH=7. Après décantation, la phase organique est séchée sur sulfate de magnésium. Après filtration et concentration à sec à l'évaporateur rotatif on obtient 1,71 g de produit sous forme de meringue qui est recristallisé dans un mélange acétate d'éthyle-éther diisopropylique (6-1 en volumes). On obtient ainsi, avec un taux de transformation de 97 %, 0,25 g de N-[hydroxy-3' chloro-2' (pyridyl-2'')-3' propanoyl] bornanesultame-10,2 contenant, d'après le spectre de résonance magnétique nucléaire du proton, un mélange 95-5 d'isomères "syn" (2'R,3'S) et "anti" (2'S,3'S).

### EXEMPLE 4

En opérant comme dans l'exemple 3 mais en utilisant 1,5 g de pyridinecarboxaldéhyde-4, on obtient, avec un taux de transformation de 85 %, 0,7 g de N-[hydroxy-3' chloro-2' (pyridyl-4)-3' propanoyl] bornanesultame-10,2 contenant, d'après le spectre de résonance magnétique nucléaire du proton, plus de 95 % d'isomère "syn" (2'R,3'S).

## Revendications

1. Procédé de préparation d'un dérivé "syn" du propanamide de formule générale : 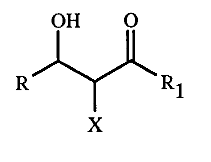 dans laquelle X représente un atome d'halogène, R représente un radical alkyle, aryle ou hétéroaryle et R₁représente le reste de l'un ou l'autre des énantiomères du camphosultame de formule : 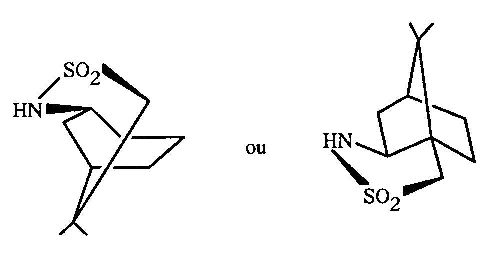 étant entendu que, selon la nature de R₁, les dérivés "syn" du propanamide ont la configuration : 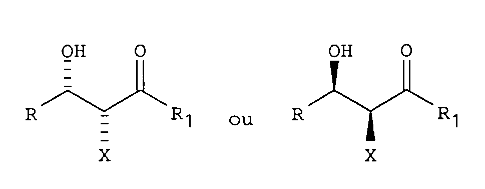 caractérisé en ce que l'on fait réagir un dérivé du camphosultame de formule générale : 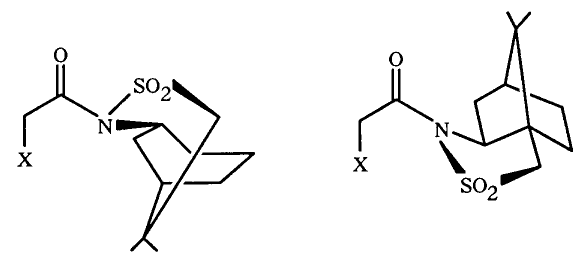 dans laquelle X est défini comme précédemment, sur un aldéhyde de formule générale :
R-CHO
dans laquelle R est défini comme précédemment en présence de chlorure de titane (TiCl₄) et d'une base organique dans un solvant organique inerte à une température comprise entre -50 et +50°C.

2. Procédé selon la revendication 1 caractérisé en ce que l'on utilise de 0,3 à 3 moles de chlorure de titane par mole de dérivé du camphosultame.

3. Procédé selon la revendication 1 caractérisé en ce que la base organique est choisie parmi les amines tertiaires aliphatiques.

4. Procédé selon la revendication 3 caractérisé en ce que la base est choisie parmi la diisopropyléthylamine et la triéthylamine.

5. Procédé selon l'une des revendications 1, 3 ou 4 caractérisé en ce que l'on utilise au moins une mole de base par mole de dérivé du camphosultame.

6. Procédé selon l'une des revendications 1 à 5 pour la préparation d'un dérivé "syn" du propanamide de formule générale : 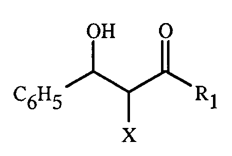 dans laquelle X et R₁ sont définis comme dans la revendication 1.

7. Procédé selon l'une des revendications 1 à 5 pour la préparation d'un dérivé "syn" du propanamide de formule générale : 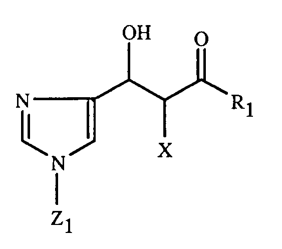 dans laquelle X et R₁ sont définis comme dans la revendication 1 et Z₁ représente le radical trityle.

## Claims

1. Process for preparing a "syn" derivative of propanamide of general formula: 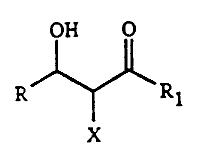 in which X represents a halogen atom, R represents an alkyl, aryl or heteroaryl radical and R₁ represents the residue of one or other of the enantiomers of camphorsultam of formula: 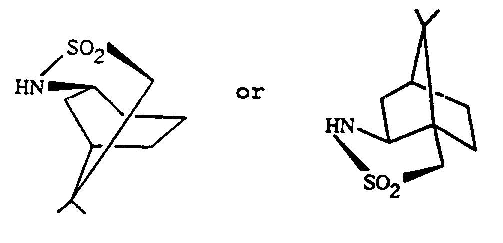 on the understanding that, according to the nature of R₁, the "syn" derivatives of propanamide have the configuration: 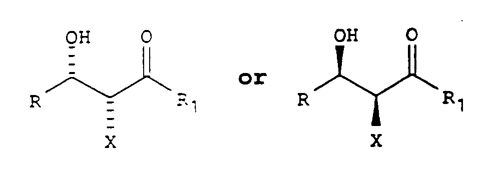 characterized in that a camphorsultam derivative of general formula: 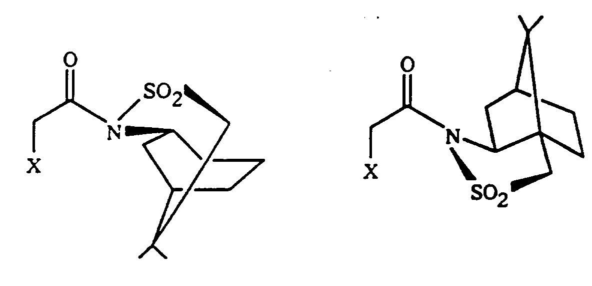 in which X is defined as above, is reacted with an aldehyde of general formula:
R-CHO
in which R is defined as above, in the presence of titanium chloride (TiCl₄) and an organic base in an inert organic solvent at a temperature of between -50 and +50°C.

2. Process according to claim 1, characterized in that from 0.3 to 3 mol of titanium chloride are used per mol of camphorsultam derivative.

3. Process according to claim 1, characterized in that the organic base is chosen from tertiary aliphatic amines.

4. Process according to claim 3, characterized in that the base is chosen from diisopropylethylamine and triethylamine.

5. Process according to one of claims 1, 3 and 4, characterized in that at least one mol of base is used per mol of camphorsultam derivative.

6. Process according to one of claims 1 to 5 for the preparation of a "syn" derivative of propanamide of general formula: 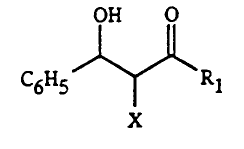 in which X and R₁ are defined as in claim 1.

7. Process according to one of claims 1 to 5 for the preparation of a "syn" derivative of propanamide of general formula: 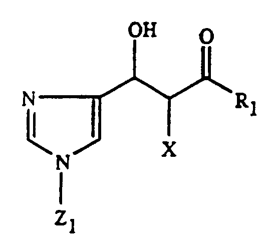 in which X and R₁ are defined as in claim 1 and Z₁ represents a trityl radical.

## Patentansprüche

1. Verfahren zur Herstellung eines "syn"-Derivats des Propanamids mit der allgemeinen Formel: 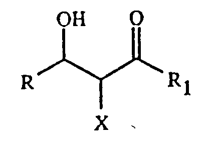 in der X für ein Halogenatom steht, R für einen Alkyl- Aryl- oder Heteroarylrest und R₁ für den Rest eines der beiden Enantiomere des Camphosultams mit der Formel stehen: 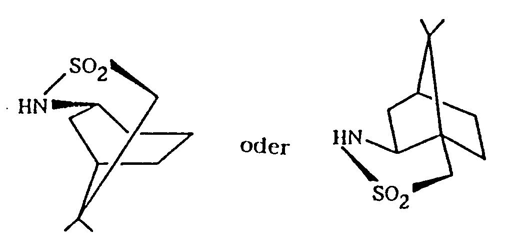 wobei gemäß der Beschaffenheit von R₁ die "syn"-Derivate des Propanamids folgende Konfiguration aufweisen: 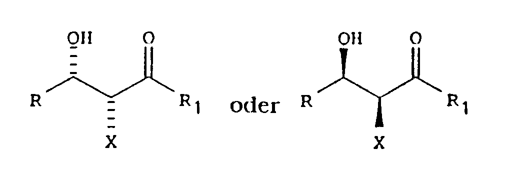 dadurch gekennzeichnet, daß man ein Derivat des Camphosultams mit der allgemeinen Formel: 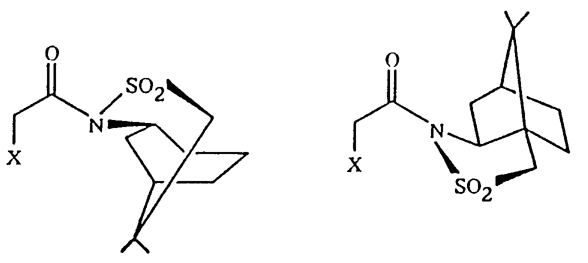 in der X wie vorher definiert ist, mit einem Aldehyd mit folgender allgemeiner Formel reagieren läßt:
R-CHO
in der R wie vorher definiert ist, in Gegenwart von Titanchlorid (TiCl₄) und einer organischen Base in einem inerten organischen Lösungsmittel bei einer Temperatur zwischen -50 und +50°C.

2. Verfahren gemäß Anspruch 1, dadurch gekennzeichnet, daß man 0,3 bis 3 Mol Titanchlorid pro Mol Camphosultamderivat verwendet.

3. Verfahren gemäß Anspruch 1, dadurch gekennzeichnet, daß die organische Base aus den aliphatischen tertiären Aminen ausgewählt ist.

4. Verfahren gemäß Anspruch 3, dadurch gekennzeichnet, daß die Base aus dem Diisopropylethylamin und dem Triethylamin ausgewählt ist.

5. Verfahren gemäß einem der Ansprüche 1, 3 oder 4, dadurch gekennzeichnet, daß man wenigstens ein Mol Base pro Mol Camphosultamderivat verwendet.

6. Verfahren gemäß einem der Ansprüche 1 bis 5 zur Herstellung eines "syn"-Derivats des Propanamids mit der allgemeinen Formel: 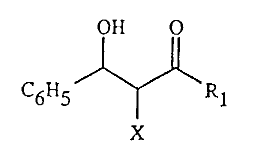 in der X und R₁ wie in Anspruch 1 definiert sind.

7. Verfahren gemäß einem der Ansprüche 1 bis 5 zur Herstellung eines "syn"-Derivats des Propanamids mit der allgemeinen Formel: 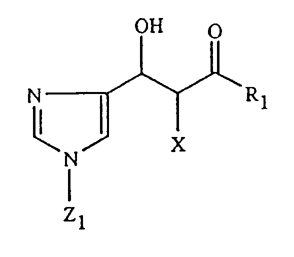 in der X und R₁ wie in Anspruch 1 definiert sind und Z₁ für den Tritylrest steht.
